# EUROPEAN PATENT SPECIFICATION

(11) **EP 2 172 225 B1**
(45) Date of publication and mention of the grant of the patent: **07.08.2019**
(21) Application number: 08790686.3
(22) Date of filing: 27.06.2008
(51) Int. Cl.: A61K 47/42, A61K 9/48, A61K 31/232, A61K 47/10, A61P 9/00

(54) **SEAMLESS CAPSULE**
NAHTLOSE KAPSEL
CAPSULE SANS SOUDURE

(30) Priority: 29.06.2007 JP 2007173198
(43) Date of publication of application: 07.04.2010
(73) Proprietor: Takeda Pharmaceutical Company Limited, Osaka 541-0045 (JP)
(72) Inventor: YOSHINARI, Tomohiro, Osaka 540-8645 (JP); UCHIYAMA, Yoshihiro, Osaka-shi Osaka 532-8686 (JP)
(74) Representative: Dossmann, Gérard
(86) International application number: PCT/JP2008/061721
(87) International publication number: WO 2009/004999

(56) References cited:
- EP-A1- 1 157 692
- WO-A1-2004/056370
- WO-A2-2005/079853
- WO-A2-2008/012329
- JP-A- H0 826 976
- JP-A- H09 155 183
- JP-A- 2004 262 774
- JP-A- 2006 512 944
- JP-A- 2007 523 904
- US-A- 5 502 077
- MASANOBU ASADA: 'Seamless Capsule no Kiso to Oyo' SHOKUHIN HYDROCOLLOIDS SYMPOSIUM vol. 16 TH, 2005, pages 34 - 38

## Description

### Technical Field

The present invention relates to a seamless capsule comprising a capsule content and a shell (film) formed by a shell (film) composition comprising gelatin and a plasticizer, which is free of an interfacial tension modifier and a gelling agent, as well as a production method thereof. In addition, the present invention relates to a seamless capsule with extremely small nonuniformity in the thickness, and without an eye or with only an extremely small eye, comprising a shell formed by a shell composition comprising gelatin and a plasticizer, which is free of an interfacial tension modifier and a gelling agent.

### (Background of the Invention)

Encapsulation techniques have been conventionally used widely in the fields of pharmaceutical products, foods, quasi-drugs, and the like. Among such capsules, seamless capsules are based on an encapsulation technique utilizing the tension produced in the oil-water interface and the gelling property of a shell substrate. While seamless capsule is one kind of soft capsules, it has been widely used in recent years in view of its characteristics such as wide range of selection of particle sizes, thickness and hardness of capsule films and capsule dissolution time, and the like.

As the shell (film)-forming material (shell (film) composition) used for forming such capsules, a material superior in the intracorporeal solubility and capable of releasing the content by being disintegrated rapidly is used. Typical examples of such material include gelatin and a material comprising a composition containing gelatin. Conventionally, seamless capsules have been obtained using an interfacial tension modifier or a gelling agent (e.g., fats and oils, phospholipid, polar organic solvent such as ethanol and the like) added to a shell composition and/or capsule content in an effort to produce seamless capsules with high quality, which contain a small eye (foam-like defect produced in the shell), and show small nonuniformity in the thickness (significantly varying shell thickness depending on the part of capsule), and the like.

Patent reference 1 describes a pharmaceutical composition for the treatment or prophylaxis of cardiovascular diseases, which comprises ω3-fatty acid in a proportion of at least 80 wt% of the total fatty acid, and (all-Z)-5,8,11,14,17-eicosapentaenoic acid (EPA) C20:5 and (all-Z)-4,7,10,13,16,19-docosahexaenoic acid (DHA) C22:6 in a relative amount of 1:2 to 2:1, constituting at least 80 wt% of the total fatty acid. Specifically, EPA ethyl ester capsule, DHA ethyl ester capsule, d-α tocopherol capsule, gelatin capsule, glycerin capsule, red iron oxide capsule and yellow iron oxide capsule are recited and soft·gelatin·capsules (1 g/1 capsules) are recited as preparation examples. These starting materials are filled in oval soft·gelatin·capsules (size 20) using a standard encapsulation machine.

Patent reference 2 describes a production method of seamless capsules comprising a shell material wrapping a filling material, which method having a step of preparing a composite nozzle apparatus having outer nozzles and inner nozzles conically arranged, a step of supplying a shell material to the aforementioned outer nozzle and a filling material to the aforementioned inner nozzle, and a step of simultaneously extruding the aforementioned shell material through the aforementioned outer nozzles and the aforementioned filling material through the aforementioned inner nozzles, wherein the aforementioned shell material passing through the aforementioned nozzles has the same flow flux as that of the aforementioned filling material passing through the aforementioned inner nozzle.

Patent reference 3 describes a production method of a seamless capsule comprising curing at least the outermost part of a droplet by contact thereof with a curing liquid, wherein the aforementioned droplet is dropped through at least a part of the aforementioned curing liquid with a helical movement.

WO 2004/056370 discloses capsule containing DHA and EPA. The pharmaceutical composition may be in the form of a soft capsule, more preferably a gelatine capsule.

Moreover, 15 to 80% of the capsule content may be DHA and EPA.

EP 1 157 692 discloses soft gelatine capsules comprising EPA/DHA.

WO 2005/079853 relates to a soft gelatine capsule containing a pharmaceutical formulation comprising at least one omega-3 polyunsaturated fatty acid in free acid form. Plasticizers like glycerine may be added to gelatine. The capsules may contain up to 30% of DHA and possibly up to 90% of EPA.

WO 2008/012329 describes polyunsaturated fatty acids like DHA or EPA in the form of capsules comprising a liquid core and a solid shell. The shell of the capsule may comprise gelatine and plasticizer such as glycerine.

None of these documents discloses a capsule having a specific minimum/maximum thickness ratio or a small diameter of eye of the shell after encapsulation.

The above-mentioned prior art references do not describe suppression of formation of the eyes and uneven thickness in a shell, and the relationship between the suppression and use of an interfacial tension modifier and a gelling agent. What should be particularly noted is the absence of description as to the stabilization of the capsule content such as a pharmaceutical ingredient to be enclosed in the capsule and the like, and increase in the amount of the capsule content (e.g., pharmaceutical ingredient).

In the meantime, use of an interfacial tension modifier and a gelling agent adversely affects the stability of the capsule content such as a pharmaceutical ingredient to be enclosed and the like, or prevents increase in the amount of the capsule content (e.g., a pharmaceutical ingredient) (increased content per unit capsule).

patent reference 1: JP-B-2810916
patent reference 2: JP-A-10-506841
patent reference 3: JP-B-3159724

### Disclosure of the Invention

### Problems to be Solved by the Invention

An object of the present invention is to provide a seamless capsule superior in the stability of the capsule content, which permits a high content of the capsule content, and a production method thereof, and furthermore, a seamless capsule free of an eye or with only an extremely small eye if present, and having extremely small nonuniformity in the thickness.

### Means of Solving the Problems

The present inventors have conducted various studies of constituent components of the shell composition and capsule content, and conditions of seamless capsule production and found that a shell (film) formed by a shell composition containing gelatin and a plasticizer, which is free of an interfacial tension modifier and a gelling agent, and a seamless capsule comprising a capsule content free of an interfacial tension modifier and a gelling agent, solve the above-mentioned problems, and established a production method thereof, which resulted in the completion of the present invention.

The present invention is specifically described as follows.
1. A method of producing the seamless capsule comprising a shell formed by a shell composition comprising gelatin and a plasticizer, which does not comprise an interfacial tension modifier chosen in the group of phospholipid, fat, oils, surfactant, polar organic solvent and a gelling agent chosen in the group of polar organic solvents and polysaccharides, and a capsule content without comprising an interfacial tension modifier chosen in the group of phospholipid, fat, oils, surfactant, polar organic solvent and a gelling agent chosen in the group of polar organic solvents and polysaccharides by a drop in oil method, wherein temperatures of the "capsule content without comprising an interfacial tension modifier and a gelling agent", the "aqueous shell composition solution formed by a shell composition comprising gelatin and a plasticizer, which does not comprise an interfacial tension modifier and a gelling agent" and the "carrier liquid" are each controlled near multiple nozzles of an apparatus used for the drop in oil method.
2. The production method of claim 1, wherein the temperature of the "capsule content without comprising an interfacial tension modifier and a gelling agent" is controlled to a value set ± 2°C within the range of 5°C - 25°C, or the temperature of the "aqueous shell composition solution formed by a shell composition comprising gelatin and a plasticizer, which does not comprise an interfacial tension modifier and a gelling agent" is controlled to a value set ± 2°C within the range of 50°C - 80°C, or the temperature of the "carrier liquid" is controlled to a value set ± 1°C within the range of 1°C - 15°C, or a difference between the temperature of the "capsule content without comprising an interfacial tension modifier and a gelling agent" and the temperature of the "aqueous shell composition solution formed by a shell composition comprising gelatin and a plasticizer, which does not comprise an interfacial tension modifier and a gelling agent" is not less than 25°C and not more than 75°C, or a difference between the temperature of the "aqueous shell composition solution formed by a shell composition comprising gelatin and a plasticizer, which does not comprise an interfacial tension modifier and a gelling agent" and the temperature of the "carrier liquid" is not less than 35°C and not more than 79°C.
3. The production method of claim 1, wherein the capsule content is a pharmaceutically active ingredient composition, optionally the pharmaceutically active ingredient composition comprises ω3-fatty acid alkyl ester, optionally the pharmaceutically active ingredient composition comprises ω3-fatty acid alkyl ester in a proportion of not less than 90%w/w.
4. The production method of claim 3, wherein the pharmaceutically active ingredient composition comprises EPA ethyl ester (eicosapentaenoic acid ethyl ester) and DHA ethyl ester (docosahexaenoic acid ethyl ester) at a total ratio of the both components of not less than 80%w/w or comprises not less than 40%w/w of EPA ethyl ester and not less than 34%w/w of DHA ethyl ester, optionally the pharmaceutically active ingredient composition comprises OMEGA-3-ACID ETHYL ESTERS 90 in the European Pharmacopoeia.
5. A seamless capsule comprising a shell formed by a shell composition comprising gelatin and a plasticizer, which does not comprise an interfacial tension modifier chosen in the group of phospholipid, fat, oils, surfactant, polar organic solvent and a gelling agent chosen in the group of polar organic solvents and polysaccharides, wherein the shell has a minimum/maximum thickness ratio of not less than 0.6, wherein the diameter of an eye of the shell after encapsulation and before drying is 1/2 or below of the average thickness of shells.
6. The seamless capsule of claim 5, wherein the shell is free of an eye.
7. The seamless capsule of claim 5, which does not comprise an interfacial tension modifier chosen in the group of phospholipid, fat, oils, surfactant, polar organic solvent and a gelling agent chosen in the group of polar organic solvents and polysaccharides, wherein the shell after encapsulation and before drying is free of an eye having a diameter exceeding 1/2 of the average thickness of shells.
8. The seamless capsule of claim 5, which comprises a capsule content free of an interfacial tension modifier chosen in the group of phospholipid, fat, oils, surfactant, polar organic solvent and a gelling agent chosen in the group of polar organic solvents and polysaccharides.
9. The seamless capsule of claim 5, wherein the capsule comprises a shell and a capsule content, wherein the capsule content is a pharmaceutically active ingredient composition, optionally the pharmaceutically active ingredient composition comprises ω3-fatty acid alkyl ester, optionally the pharmaceutically active ingredient composition comprises ω3-fatty acid alkyl ester in a proportion of not less than 90%w/w.
10. The seamless capsule of claim 9, wherein the pharmaceutically active ingredient composition comprises EPA ethyl ester (eicosapentaenoic acid ethyl ester) and DHA ethyl ester (docosahexaenoic acid ethyl ester) at a total ratio of the both components of not less than 80%w/w or comprises not less than 40%w/w of EPA ethyl ester and not less than 34%w/w of DHA ethyl ester, optionally the pharmaceutically active ingredient composition comprises OMEGA-3-ACID ETHYL ESTERS 90 in the European Pharmacopoeia.

### Effect of the Invention

Since the seamless capsule of the present invention does not contain an interfacial tension modifier chosen in the group of phospholipid, fat, oils, surfactant, polar organic solvent and a gelling agent chosen in the group of polar organic solvents and polysaccharides, it is superior in the stability of the capsule content, and permits a high content of a capsule content. Moreover, the present invention can provide a high quality seamless capsule free of an eye or with an extremely small eye even if present, which shows extremely small nonuniformity in the thickness.

Since the content can be high, the unit size of a preparation can be minimized, and highly easy administration and small space utility during storage can be realized. Moreover, the stability during preservation can be improved, thus enabling extension of a use-by date.

### Brief Description Of The Drawings

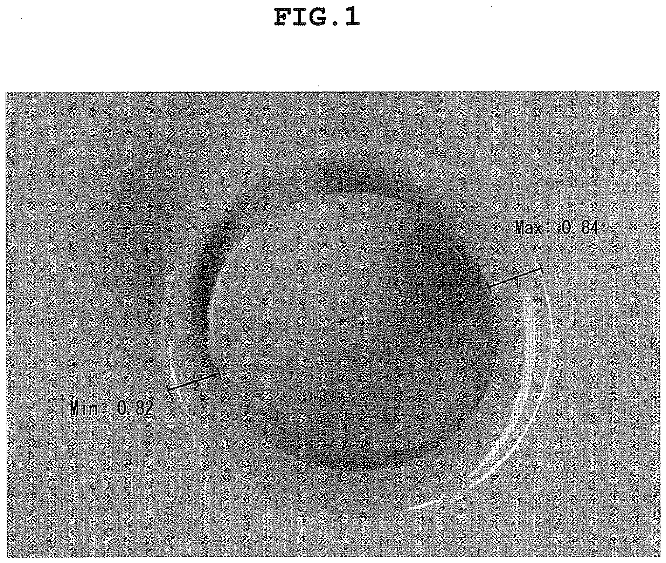

[Fig. 1] The seamless capsule of the present invention.

### (Detailed Description Of The Invention)

The present invention is explained in detail in the following by referring to specific examples.

### (Composition of shell composition)

The shell (film) covering the capsule content of the seamless capsule of the present invention is formed by a shell composition comprising gelatin and a plasticizer, which does not comprise an interfacial tension modifier chosen in the group of phospholipid, fat, oils, surfactant, polar organic solvent and a gelling agent chosen in the group of polar organic solvents and polysaccharides.

As used herein, the "gelatin" is, for example, one generally used for the production of a seamless capsule, such as the pharmaceutical gelatin defined in the Japanese Pharmacopoeia Fifteenth Edition. The gelatin here is a concept including, for example, modified gelatin such as succinylated gelatin and the like.

Particularly, gelatin preferable for practicing the present invention includes those having the following properties.
1) The jelly strength of gelatin (measured based on the method described in JIS) is 200 - 300 g, preferably 240 - 300 g, more preferably 240 - 280 g.
2) The viscosity of gelatin (measured based on the method described in JIS) is 2 - 6 mPa·s, preferably 3 - 6 mPa·s, more preferably 3 - 5 mPa·s.
3) The gelatin is derived from pigskin.

For the production of the seamless capsule of the present invention, one or more kinds of gelatin may be combined. In this case, the mixed gelatin preferably has the jelly strength of the above-mentioned 1) and/or the gelatin viscosity of the above-mentioned 2).

Most preferred one satisfies one or more of the above-mentioned conditions.

As the "plasticizer" here, those generally used for the production of seamless capsules can be mentioned and, for example, polyvalent alcohols such as glycerin, ethylene glycol, polyethylene glycol, propylene glycol, polypropylene glycol and the like, and sugar alcohols such as sorbitol and the like are preferable. These plasticizers are used in a combination of one or more kinds thereof.

Among those, preferred for practicing the present invention are glycerin and sorbitol.

It is also preferable to use them in a mixture. In this case, the weight ratio of glycerin and sorbitol is preferably within the range of 1:5 - 5:1, particularly preferably 1:3 - 3:1, for practicing the present invention.

For practicing the present invention, the shell composition preferably comprises gelatin and a plasticizer at a weight ratio within the range of 10:1 - 1:10, particularly preferably 10:1 - 1:1.

### (Capsule content)

The content to be encapsulated in the seamless capsule of the present invention is not particularly limited as long as it does not comprise an interfacial tension modifier chosen in the group of phospholipid, fat, oils, surfactant, polar organic solvent and gelling agent chosen in the group of polar organic solvents and polysaccharides.

As the content, a material desired for the object is used. For example, a pharmaceutically active ingredient, food, flavor, seed, microorganism, plant cell, plant tissue, animal cell, animal tissue (hereinafter to be referred to as the main component) and the like can be mentioned. The capsule content may be the main component itself or a composition containing the main component and other additive. The content of the main component, and the kind and content of the additive, etc. in the seamless capsule can be appropriately determined according to the object of use thereof. The present invention is explained in more detail in the following by taking a "pharmaceutically active ingredient" as an example of the capsule content.

While the pharmaceutically active ingredient to be included in the seamless capsule is not particularly limited, for example, a compound having easily oxidizable properties and an oil compound are preferable.

One example of such pharmaceutically active ingredient is ω3-fatty acid alkyl ester (e.g., ω3-fatty acid C₁₋₆ alkyl ester; preferably ω3-fatty acid ethyl ester). Here, examples of the "ω3-fatty acid" of ω3-fatty acid alkyl ester include C₁₈₋₂₂ ω3-fatty acid (e.g., octadecatrienoic acid, octadecatetraenoic acid, eicosatetraenoic acid, eicosapentaenoic acid, heneicosapentaenoic acid, docosapentaenoic acid, docosahexaenoic acid).

Specific examples of the ω3-fatty acid ethyl ester include octadecatrienoic acid ethyl ester, octadecatetraenoic acid ethyl ester, eicosatetraenoic acid ethyl ester, eicosapentaenoic acid (EPA) ethyl ester, heneicosapentaenoic acid ethyl ester, docosapentaenoic acid ethyl ester, docosahexaenoic acid (DHA) ethyl ester, and a mixture of two or more kinds thereof (e.g., OMEGA-3-ACID ETHYL ESTERS 90 in the European Pharmacopoeia, which is useful for the treatment of hyperlipidemia (e.g., hypercholesterolemia, hyper-LDL cholesterolemia, low-HDL cholesterolemia, hypertriglyceride(TG)mia) and the like, prevention of cardiovascular event, and the like).

When a pharmaceutically active ingredient is encapsulated in a seamless capsule, it is included in the form of a "pharmaceutically active ingredient composition". Here, the "pharmaceutically active ingredient composition" encompasses a pharmaceutically active ingredient itself and a mixture of a pharmaceutically active ingredient and additive(s) generally used in the field of pharmaceutical products.

Examples of the specific pharmaceutically active ingredient composition mentioned above are
1) a composition comprising ω3-fatty acid alkyl ester at not less than 90%w/w (e.g., 90 - 100%w/w);
2) the composition of the above-mentioned 1) comprising ω3-fatty acid alkyl ester at not less than 90%w/w (e.g., 90 - 100%w/w), wherein EPA ethyl ester and/or DHA ethyl ester are/is the main component(s);
3) the composition of the above-mentioned 2) comprising both components of EPA ethyl ester and DHA ethyl ester, wherein the total content ratio of the both components is not less than 80%w/w (e.g., 80 - 100%w/w; preferably 80 - 90%w/w) (here, the composition may contain ω3-fatty acid alkyl ester other than EPA ethyl ester and DHA ethyl ester);
4) the composition of the above-mentioned 3) comprising EPA ethyl ester in not less than 40%w/w (e.g., 40 - 66%w/w; preferably 40 - 50%w/w) and DHA ethyl ester in not less than 34%w/w (e.g., 34 - 60%w/w; preferably 34 - 45%w/w) wherein the total content ratio of the EPA ethyl ester and the DHA ethyl ester is not less than 80%w/w, and the composition may contain ω3-fatty acid alkyl ester other than the EPA ethyl ester and DHA ethyl ester;
5) the composition of the above-mentioned 4) which is OMEGA-3-ACID ETHYL ESTERS 90 defined by the European Pharmacopoeia; wherein %w/w is the content ratio of the pharmaceutically active ingredient relative to the total weight of the capsule content.

The above-mentioned pharmaceutically active ingredient compositions 1) - 5) may be constituted by ω3-fatty acid alkyl ester alone, which is a pharmaceutically active ingredient, or may further contain additive(s) other than the pharmaceutically active ingredient.

As such additive, those mentioned below can be mentioned. Of those, a stabilizer (preferably, α-tocopherol) is preferable. When a stabilizer is to be used, its content in the pharmaceutically active ingredient composition is 0.3 - 0.5%w/w.

The pharmaceutically active ingredient composition can be used for the production of a seamless capsule in the form of a solution, emulsion, suspension or other liquid. When the pharmaceutically active ingredient itself is a liquid, it can also be used as it is.

Here, the weight ratio of the weight of the pharmaceutically active ingredient composition and the "total weight of the gelatin and a plasticizer" is generally 10:1 - 1:10, preferably 10:1 - 1:3.

The weight ratio of the weight of the shell and the weight of the capsule content is generally 10:1 - 1:10, preferably 3:1 - 1:10.

### (Interfacial tension modifier and gelling agent)

The seamless capsule of the present invention is constituted by a shell and a capsule content.

In the seamless capsule of the present invention, a shell composition is characteristically free of both an interfacial tension modifier chosen in the group of phospholipid, fat, oils, surfactant, polar organic solvent and a gelling agent chosen in the group of polar organic solvents and polysaccharides conventionally widely used for the production of seamless capsules. Due to the absence of these components in a seamless capsule, the defects of the Prior Art can be avoided. The capsule content does not comprise both an interfacial tension modifier chosen in the group of phospholipid, fat, oils, surfactant, polar organic solvent and a gelling agent chosen in the group of polar organic solvents and polysaccharides in the same manner. Due to the absence, the defects of the Prior Art can also be avoided.

Here, the "interfacial tension modifier" i.e. phospholipid, fats and oils, surfactant, polar organic solvents such as alcohol, refers to a substance that changes the tension produced in the oil-water interface between the capsule content and the aqueous shell composition solution.

Here, the "gelling agent" i.e. polar organic solvents such as alcohol and polysaccharides, refers to a substance that promotes gelling of an aqueous shell composition solution by cooling.

Here, being "free of an interfacial tension modifier and a gelling agent" means
1) being completely free of both components in a seamless capsule, or
2) even when both components or either component is contained in a seamless capsule, the content thereof being "insufficient to significantly change the interfacial tension" or "insufficient to significantly shorten the gelling time.

As used herein, the amount "to significantly change the interfacial tension" is, for example, an amount causing a change of not less than 5 mN/m, or an amount causing a change of not less than 10%, as compared to no addition of an "interfacial tension modifier", when a tension produced in the oil-water interface between an aqueous gelatin solution (21.25%w/w, 50°C) and a content liquid (24°C) is measured. The interfacial tension is measured by, for example, a pendant drop method using FTA200 of First Ten Angstroms, Inc.

The "amount to significantly shorten the gelling time" is, for example, an amount that shortens the time necessary for gelling upon dropwise addition of an aqueous gelatin solution (21.25%w/w) heated to 55°C to MCT oil at 4°C, namely, the time necessary for reaching 50% level of elasticity of an aqueous gelatin solution at 4°C, by not less than 10%, as compared to no addition of a "gelling agent".

In other words, being "free of an interfacial tension modifier and a "gelling agent" means that an "interfacial tension modifier" chosen in the group of phospholipid, fat, oils, surfactant, polar organic solvent is not present and a "gelling agent" chosen in the group of polar organic solvents and polysaccharides is not present, either (including being substantially not contained (i.e., substantial absence)). Here, "substantial absence" of an interfacial tension modifier means possible presence thereof in "an amount insufficient to significantly change the interfacial tension", and that of a gelling agent means possible presence thereof in "an amount insufficient to significantly shorten the gelling time".

Accordingly, being "free of an interfacial tension modifier chosen in the group of phospholipid, fat, oils, surfactant, polar organic solvent and a gelling agent chosen in the group of polar organic solvents and polysaccharides includes the following embodiments:
1) the seamless capsule of the present invention is completely free of both an interfacial tension modifier chosen in the group of phospholipid, fat, oils, surfactant, polar organic solvent and a gelling agent chosen in the group of polar organic solvents and polysaccharides;
2-1) the seamless capsule of the present invention is substantially free of both an interfacial tension modifier chosen in the group of phospholipid, fat, oils, surfactant, polar organic solvent and a gelling agent chosen in the group of polar organic solvents and polysaccharides;
2-2) the seamless capsule of the present invention is substantially free of an interfacial tension modifier chosen in the group of phospholipid, fat, oils, surfactant, polar organic solvent and completely free of a gelling agent chosen in the group of polar organic solvents and polysaccharides; and
2-3) the seamless capsule of the present invention is completely free of an interfacial tension modifier chosen in the group of phospholipid, fat, oils, surfactant, polar organic solvent and substantially free of a gelling agent chosen in the group of polar organic solvents and polysaccharides.

The seamless capsule of the present invention may contain components other than the "interfacial tension modifier" chosen in the group of phospholipid, fat, oils, surfactant, polar organic solvent and "gelling agent" chosen in the group of polar organic solvents and polysaccharides when desired and, for example, may contain a colorant. A colorant to be contained in the composition is not particularly limited and a desired colorant is used as necessary. For example, food colors such as yellow dye No. 5, red dye No. 2, blue dye No. 2 and the like; β carotene; food lake colors; diiron trioxide, yellow ferric oxide; and the like can be mentioned.

When desired, moreover, the seamless capsule of the present invention may contain components such as monosaccharides (e.g., pentose such as arabinose, xylose, ribose, 2-deoxyribose and the like; hexose such as glucose, fructose, galactose, mannose, sorbose, rhamnose, fucose and the like), disaccharides (e.g., malt sugar, cellobiose, α,α-trehalose, lactose, sucrose), celluloses (e.g., crystalline cellulose (including microcrystalline cellulose)), inorganic products (e.g., anhydrous calcium phosphate, precipitated calcium carbonate, calcium silicate) and the like.

When any of the above-mentioned components has an effect of changing the interfacial tension and/or an effect of shortening the gelling time, the seamless capsule of the present invention may even contain a component having such effect in an amount insufficient to significantly change the interfacial tension and an amount insufficient to significantly shorten the gelling time.

The seamless capsule of the present invention may further contain, in addition to the above-mentioned components, additive(s) generally used in the field of the pharmaceutical products. Examples of such additive include inactive diluents (e.g., medium-chain triglyceride (MCT) oil, olive oil, soybean oil, corn oil, water, ethanol, a mixture thereof); pH adjusters (e.g., citrate, phosphate, carbonate, tartrate, fumarate, acetate, amino acid salt); surfactants (e.g., sodium lauryl sulfate, polysorbate 80, polyoxyethylene(160)polyoxypropylene(30)glycol); stabilizers (e.g., α-tocopherol, sodium tetraedetate, amide nicotinic acid, cyclodextrins); acidulants (e.g., ascorbic acid, citric acid, tartaric acid, malic acid); flavors (e.g., menthol, peppermint oil, lemon oil, vanillin, blueberry flavor); fluidizers (e.g., light anhydrous silicic acid, hydrated silicon dioxide, talc); and the like.

When any of the above-mentioned additives has an effect of changing the interfacial tension and/or an effect of shortening the gelling time, the seamless capsule of the present invention may even contain an additive having such effect in an amount insufficient to significantly change the interfacial tension and an amount insufficient to significantly shorten the gelling time.

Each component of the shell composition above is, for example, blended and dissolved in an aqueous medium and used for the production of a seamless capsule (referred to as an "aqueous shell composition solution").

The concentration of the components (component concentration) other than water in the "aqueous shell composition solution" is preferably 15 - 35%w/w. When a low viscosity gelatin is used, the component concentration can be increased, which in turn shortens the drying time after production of a seamless capsule. Here, the low viscosity means 2 - 4 mPa·s.

### (Carrier liquid)

For production of the seamless capsule of the present invention, a "carrier liquid" (e.g., MCT oil, etc.) generally used in this field can be employed.

Examples of the "carrier liquid" generally used in this field include oily substrates such as medium-chain triglyceride (MCT) oil, olive oil, soybean oil, corn oil and the like.

### (Preparation of seamless capsule)

The seamless capsule of the present invention is prepared using the above-mentioned "aqueous shell composition solution" and "capsule content", and a capsule production apparatus generally used for the production of seamless capsules (e.g., "SPHEREX" manufactured by Freund Corporation) and by a dropping immersed in a carrier liquid using multiple nozzles for the both components.

To be more specific, a capsule content flows out from an inner nozzle of multiple nozzles into a carrier liquid and an aqueous shell composition solution from an outer nozzle thereof into the carrier liquid at constant rates, a fluid of these two layers is cut at given intervals to form droplets with an interfacial tension, and the outer shell layer is gelled by cooling to give seamless capsules. The obtained seamless capsules are cooled for a given time and dried to yield products.

However, production of the seamless capsule of the present invention is not limited to such production method and can also be produced by other method known in this field.

The size of the seamless capsule of the present invention can be appropriately adjusted by a known method. A capsule having a diameter within the range of 1 - 10 mm is preferable, and a capsule having a diameter within the range of 2 - 6 mm is more preferable.

For production of the seamless capsule of the present invention, it is preferable to appropriately control the temperature conditions at the capsule formation point (near multiple nozzles) during the production.

When the main component of a capsule content shows an excessively low or excessively high interfacial tension at a temperature near 25°C, as compared to appropriate interfacial tension, (especially when the content of the main component showing an excessively low or excessively high interfacial tension in the capsule content is high), it is particularly preferable for the production of a high quality seamless capsule to appropriately control the temperature.

In the present invention, the appropriate interfacial tension is, for example, 15 - 50 mN/m in the interface with an aqueous gelatin solution (21.25%w/w, 50°C). An interfacial tension below this level can be classified as being "excessively low", and an interfacial tension above this level can be classified as being "excessively high". The interfacial tension can be measured, for example, by the aforementioned method. This numerical value is one index that varies within a reasonable range depending on the capsule content to which the present invention is applied. Those of ordinary skill in the art can consider the necessity of temperature control as appropriate at specific situations where the present invention is applied.

In a preferable embodiment, a seamless capsule is produced while keeping various conditions below wherein, near multiple nozzles of an apparatus for seamless capsule production,
1-1) the temperature of the "capsule content" is controlled to a value set ± 2°C within the range of 5 - 25°C;
2-1) the temperature of the "aqueous shell composition solution" is controlled to a value set ± 2°C within the range of 50 - 80°C;
3-1) the temperature of the "carrier liquid" is controlled to a value set ± 1°C within the range of 1 - 15°C;
4-1) the difference between the temperature of the "capsule content" and the temperature of the "aqueous shell composition solution" is not less than 25°C and not more than 75°C; and
5-1) the difference between the temperature of the "aqueous shell composition solution" and the temperature of the "carrier liquid" is not less than 35°C and not more than 79°C.

While a seamless capsule with sufficiently high quality can be obtained under the above temperature conditions, for production of a seamless capsule having still higher quality, a seamless capsule is produced while keeping various conditions below wherein
1-2) the temperature of the "capsule content" is controlled to a value set ± 2°C (preferably ± 1°C) within the range of 12°C - 22°C;
2-2) the temperature of the "an aqueous shell composition solution" is controlled to a value set ± 2°C (preferably ± 1°C) within the range of 60°C - 70°C;
3-2) the temperature of the "carrier liquid" is controlled to a value set ± 1°C (preferably ±0.5°C) within the range of 3 - 11°C;
4-2) the difference between the temperature of the "capsule content" and the temperature of the "aqueous shell composition solution" is not less than 38°C and not more than 58°C; and
5-2) the difference between the temperature of the "aqueous shell composition solution" and the temperature of the "carrier liquid" is not less than 49°C and not more than 67°C.

The aforementioned temperature conditions can be appropriately determined by those of ordinary skill in the art according to the level of quality required of a seamless capsule.

When producing the seamless capsule of the present invention, it is particularly advantageous to satisfy all the above conditions 1-1) to 5-1).

While the above-mentioned temperature control can be easily performed by those of ordinary skill in the art by, for example, combining PID control and feedback control, the control method is not limited thereto.

The seamless capsule of the present invention comprises a shell formed by a shell composition containing gelatin and a plasticizer, but free of an interfacial tension modifier chosen in the group of phospholipid, fat, oils, surfactant, polar organic solvent and a gelling agent chosen in the group of polar organic solvents and polysaccharides. The seamless capsule of the present invention preferably does not comprise an interfacial tension modifier chosen in the group of phospholipid, fat, oils, surfactant, polar organic solvent and a gelling agent chosen in the group of polar organic solvents and polysaccharides in a capsule content thereof, and shows a shell thickness minimum value/maximum value ratio of one capsule of not less than 0.6 immediately after encapsulation (after encapsulation and before drying). Namely, the seamless capsule is a novel capsule since it shows an extremely small nonuniformity in the thickness even though the shell does not contain an interfacial tension modifier and a gelling agent, and the capsule content preferably does not contain these components.

Specifically, the level of uneven thickness of the seamless capsule of the present invention by microscopic observation immediately after encapsulation corresponds to a minimum/maximum ratio of the shell thickness before drying of not less than 0.6, more preferably not less than 0.8, still more preferably not less than 0.9. The thickness can be measured by a general image analysis method. When the shell thickness minimum/maximum ratio is less than 0.6, defectively, the uneven thickness increases, the shell is cracked during drying and storage after drying, and the capsule content leaks out.

Since shells shrink uniformly during drying, the shell thickness minimum/maximum ratio before drying is maintained even after drying.

In addition, the seamless capsule of the present invention comprises a shell formed by a shell composition containing gelatin and a plasticizer, but free of an interfacial tension modifier chosen in the group of phospholipid, fat, oils, surfactant, polar organic solvent and a gelling agent chosen in the group of polar organic solvents and polysaccharides. Preferably, its capsule content is free of an interfacial tension modifier chosen in the group of phospholipid, fat, oils, surfactant, polar organic solvent and a gelling agent chosen in the group of polar organic solvents and polysaccharides, and the capsule is free of an eye, or with an extremely small eye even if present. When an eye is present, the diameter of the eye is not more than 1/2, more preferably not more than 1/4, still more preferably not more than 1/8, of the average thickness ((maximum + minimum)/2) of the shell before drying, by microscopic observation immediately after encapsulation and before drying. Particularly, the present invention is a seamless capsule comprising a shell formed by a shell composition containing gelatin and a plasticizer, but free of an interfacial tension modifier chosen in the group of phospholipid, fat, oils, surfactant, polar organic solvent and a gelling agent chosen in the group of polar organic solvents and polysaccharides, which does not contain an eye having a diameter exceeding 1/2 of the average thickness of the shell when measured after encapsulation and before drying, preferably a seamless capsule comprising a capsule content free of an interfacial tension modifier chosen in the group of phospholipid, fat, oils, surfactant, polar organic solvent and a gelling agent chosen in the group of polar organic solvents and polysaccharides. When the eye has a diameter exceeding 1/2 of the average thickness of the shell ((maximum + minimum)/2), defects such as cracks developed in the shell during drying and storage after drying and leakage of the capsule content occur.

Here, the eye refers to a spherical foam-like defect produced in the shell, and the foam may contain a capsule content. While the eye is generally a sphere with small distortion, it may be a distorted sphere. In a non-spherical eye, the "eye diameter" refers to the diameter in the maximum direction. A seamless capsule may have multiple eyes, and each diameter is within the above-mentioned range. The thickness and eye diameter can be measured by general image analysis methods as in the measurement of uneven thickness.

In the present specification, when an eye with a diameter of 1/2 or less of the average thickness of the shell is present in a shell, the shell may sometimes be expressed to have "no eye".

The seamless capsule of the present invention comprising a pharmaceutically active ingredient as its content can be safely administered to mammals (e.g., mouse, rat, rabbit, cat, dog, bovine, horse, monkey, human).

In this embodiment, the dose of the seamless capsule of the present invention need only be an effective amount of each pharmaceutically active ingredient to be contained in the capsule. While the administration frequency of the seamless capsule of the present invention to the aforementioned mammals in one day varies depending on the properties of the pharmaceutically active ingredient to be contained, it is typically 1 - 3 times a day.

As a specific example, the effective amount of OMEGA-3-ACID ETHYL ESTERS 90 is 100 - 6000 mg/day for an adult (body weight 60 kg).

Specific examples of a particularly preferable seamless capsule of the present invention include the following:
1) a seamless capsule containing gelatin, and glycerin and sorbitol as plasticizers, and 25 mg of OMEGA-3-ACID ETHYL ESTERS 90 as a capsule content per capsule;
2) a seamless capsule containing gelatin, and glycerin and sorbitol as plasticizers, and 50 mg of OMEGA-3-ACID ETHYL ESTERS 90 as a capsule content per capsule;
3) a seamless capsule containing gelatin, and glycerin and sorbitol as plasticizers, and 100 mg of OMEGA-3-ACID ETHYL ESTERS 90 as a capsule content per capsule;
4) a seamless capsule containing gelatin, and glycerin and sorbitol as plasticizers, and 200 mg of OMEGA-3-ACID ETHYL ESTERS 90 as a capsule content per capsule.

### Examples

The present invention is explained in more detail in the following Examples and Experimental Examples which are not to be construed as limitative.

The various gelatins to be used in the following Examples are commercially available, or even if they are not commercially available, they can be easily obtained by requesting gelatin manufacturers to produce them.

As preparation additives, those listed in the Japanese Pharmacopoeia Fifteenth Edition, the Japanese Pharmacopoeia Pharmaceutical Codex or Pharmaceutical Excipients 2003 were used.

### Example 1

Gelatin 1 (jelly strength 245 g, viscosity 4.3 mPa·s, 3158 g), gelatin 2 (jelly strength 297 g, viscosity 5.3 mPa·s, 1579 g), concentrated glycerin (559.0 g), sorbitol solution (399.2 g, solid content: 299.4 g) and yellow dye No. 5 (1.17 g) were added to purified water (16610 g) heated to 52°C. The mixture was dissolved, and degassed under reduced pressure (aqueous shell composition solution). The composition ratio when OMEGA-3-ACID ETHYL ESTERS 90 is 1000.00 parts by weight is shown in Table 1.

An apparatus for seamless capsule production (SPHEREX, manufactured by Freund Corporation) was used to prepare seamless capsules. The temperature of "OMEGA-3-ACID ETHYL ESTERS 90" (capsule content) near the nozzle was controlled to 16.3 - 18.9°C, aqueous shell solution was controlled to 67.8 - 68.3°C, MCT oil, which was used as a carrier liquid, was controlled to 5.9 - 7.1°C, and the mixture was encapsulated at a rate of 25 capsules per second.

The encapsulation step could be constantly operated stably by strictly controlling the temperatures.

The obtained capsules were cooled in a refrigerator (5°C, 14 hr), and dried in a drier until the water activity reached not more than 0.2. Then the carrier liquid on the capsule surface was wiped off to give seamless capsules containing 25 mg of OMEGA-3-ACID ETHYL ESTERS 90 per capsule.

The size of the obtained capsules was about 4 mmφ (variation within the range of 3.5 - 4.5 mmφ) in the center value.

**[Table 1]**

| composition ratio | (-) |
|---|---|
| OMEGA-3-ACID ETHYL ESTERS 90 | 1000.00 |
| gelatin 1 | 242.94 |
| gelatin 2 | 121.47 |
| concentrated glycerin | 43.00 |
| sorbitol | 21.50 |
| yellow dye No. 5 | 0.090 |
| purified water | 1287.00 |

### Example 2

Gelatin 3 (jelly strength 255 g, viscosity 3.5 mPa·s, 5096.7 g), concentrated glycerin (601.4 g), sorbitol solution (429.2 g, solid content: 300.4 g) and yellow dye No. 5 (1.26 g) were added to purified water (13999.9 g) heated to 55°C. The mixture was dissolved, and degassed under reduced pressure (aqueous shell composition solution). The composition ratio when OMEGA-3-ACID ETHYL ESTERS 90 is 1000.00 parts by weight is shown in Table 2.

An apparatus for seamless capsule production (SPHEREX, manufactured by Freund Corporation) was used to prepare seamless capsules. The temperature of "OMEGA-3-ACID ETHYL ESTERS 90" (capsule content) near the nozzle was controlled to 16.3 - 16.7°C, and the aqueous shell solution was controlled to 70.3 - 70.4°C. MCT oil was used as a carrier liquid and the temperature thereof was controlled to 6.8 - 7.1°C, and the mixture was encapsulated at 25 capsules per second.

The encapsulation step could be constantly operated stably by strictly controlling the temperatures.

The obtained capsules were cooled in a refrigerator (4°C, 28 hr), and dried in a drier until the water activity reached not more than 0.2. Then the carrier liquid on the capsule surface was wiped off to give seamless capsules containing 25 mg of OMEGA-3-ACID ETHYL ESTERS 90 per capsule.

The size of the obtained capsules was about 4 mmφ (variation within the range of 3.5 - 4.5 mmφ) in the center value.

**[Table 2]**

| composition ratio | (-) |
|---|---|
| OMEGA-3-ACID ETHYL ESTERS 90 | 1000.00 |
| gelatin 3 | 364.41 |
| concentrated glycerin | 43.00 |
| sorbitol | 21.50 |
| yellow dye No. 5 | 0.090 |
| purified water | 1287.00 |

### Example 3

Gelatin 1 (jelly strength 245 g, viscosity 4.3 mPa·s, 3158 g), gelatin 2 (jelly strength 297 g, viscosity 5.3 mPa·s, 1579 g), concentrated glycerin (559.0 g), sorbitol solution (399.2 g, solid content: 299.4 g), and yellow dye No. 5 (1.17 g) were added to purified water (16610 g) heated to 52°C. The mixture was dissolved, and degassed under reduced pressure (aqueous shell composition solution). The composition ratio when OMEGA-3-ACID ETHYL ESTERS 90 is 1000.00 parts by weight is shown in Table 3.

An apparatus for seamless capsule production (SPHEREX, manufactured by Freund Corporation) was used to prepare seamless capsules. The temperature of "OMEGA-3-ACID ETHYL ESTERS 90" (capsule content) near the nozzle was controlled to 17.7 - 18.8°C, and the aqueous shell solution was controlled to 67.5 - 68.1°C. MCT oil was used as a carrier liquid and the temperature thereof was controlled to 6.4 - 6.8°C, and the mixture was encapsulated at a rate of 25 capsules per second.

The encapsulation step could be constantly operated stably by strictly controlling the temperatures.

The obtained capsules were cooled in a refrigerator (5°C, 14 hr), and dried in a drier until the water activity reached not more than 0.2. Then the carrier liquid on the capsule surface was wiped off to give seamless capsules containing 25 mg of OMEGA-3-ACID ETHYL ESTERS 90 per capsule.

The size of the obtained capsules was about 4 mmφ (variation within the range of 3.5 - 4.5 mmφ) in the center value.

**[Table 3]**

| composition ratio | (-) |
|---|---|
| OMEGA-3-ACID ETHYL ESTERS 90 | 1000.00 |
| gelatin 1 | 242.94 |
| gelatin 2 | 121.47 |
| concentrated glycerin | 43.00 |
| sorbitol | 21.50 |
| yellow dye No. 5 | 0.090 |
| purified water | 1287.00 |

### Example 4

Gelatin 4 (jelly strength 264 g, viscosity 4.2 mPa·s, 3398 g), concentrated glycerin (401 g), sorbitol solution (286 g, solid content: 200.2 g), and yellow dye No. 5 (0.84 g) were added to purified water (15914 g) heated to 55°C. The mixture was dissolved, and degassed under reduced pressure (aqueous shell composition solution). The composition ratio when OMEGA-3-ACID ETHYL ESTERS 90 is 1000.00 parts by weight is shown in Table 4.

An apparatus for seamless capsule production (SPHEREX, manufactured by Freund Corporation) was used to prepare seamless capsules. The temperature of "OMEGA-3-ACID ETHYL ESTERS 90" (capsule content) near the nozzle was controlled to 14.7°C, and the aqueous shell solution was controlled to 56.7°C, MCT oil was used as a carrier liquid and the temperature thereof was controlled to 4.4°C, and the mixture was encapsulated at 7 capsules per second.

The encapsulation step could be constantly operated stably by strictly controlling the temperatures.

The obtained capsules were cooled in a refrigerator (4°C, 21 hr), and dried in a drier until the water activity reached not more than 0.2. Then the carrier liquid on the capsule surface was wiped off to give seamless capsules containing 200 mg of OMEGA-3-ACID ETHYL ESTERS 90 per capsule.

The size of the obtained capsules was about 8 mmφ (variation within the range of 7.5 - 8.5 mmφ) in the center value.

**[Table 4]**

| composition ratio | (-) |
|---|---|
| OMEGA-3-ACID ETYYL ESTERS 90 | 1000.00 |
| gelatin 4 | 195.38 |
| concentrated glycerin | 23.06 |
| sorbitol | 11.51 |
| yellow dye No. 5 | 0.048 |
| purified water | 919.96 |

### Example 5

Gelatin 4 (jelly strength 264 g, viscosity 4.2 mPa·s, 9568.125 g), concentrated glycerin (1128.75 g) and sorbitol solution (806.1 g, solid content: 564.3 g) were added to purified water (33541.5 g) heated to 55°C. The mixture was dissolved, and degassed under reduced pressure (aqueous shell composition solution). The composition ratio when OMEGA-3-ACID ETHYL ESTERS 90 is 1000.00 parts by weight is shown in Table 5.

An apparatus for seamless capsule production (manufactured by Fuji Capsule Co., Ltd.) was used to prepare seamless capsules. The temperature of "OMEGA-3-ACID ETHYL ESTERS 90" (capsule content) near the nozzle was controlled to 22.8 - 23.2°C, and the aqueous shell solution was controlled to 69.9 - 70.3°C. MCT oil was used as a carrier liquid and the temperature thereof was controlled to 6.4 - 6.7°C, and the mixture was encapsulated at 25 capsules per second.

The encapsulation step could be constantly operated stably by strictly controlling the temperatures.

The obtained capsules were cooled in a refrigerator (5°C, 15 hr), and dried in a drier until the water activity reached not more than 0.2. Then the carrier liquid on the capsule surface was wiped off to give seamless capsules containing 25 mg of OMEGA-3-ACID ETHYL ESTERS 90 per capsule.

The size of the obtained capsules was about 4 mmφ (variation within the range of 3.5 - 4.5 mmφ) in the center value.

**[Table 5]**

| composition ratio | (-) |
|---|---|
| OMEGA-3-ACID ETYYL ESTERS 90 | 1000.00 |
| gelatin 4 | 364.50 |
| concentrated glycerin | 43.00 |
| sorbitol | 21.50 |
| purified water | 1287.00 |

### Example 6

Using the seamless capsules obtained in Example 5, aromatized seamless capsules were produced. The composition ratio when OMEGA-3-ACID ETHYL ESTERS 90 is 1000.00 parts by weight is shown in Table 6.

Ethyl alcohol (22.4 g) and blueberry flavor (5.6 g) were mixed to give a flavoring liquid. The flavoring liquid (1.47 g per 300 g of seamless capsules) was dispersed on seamless capsules obtained in Example 5 to aromatize them using a tumbler coating machine (HICOATER: manufactured by Freund Corporation). After aromatization, the ethyl alcohol in the seamless capsules was removed using a tumbler coating machine (HICOATER: manufactured by Freund Corporation) until it reached not more than 5000 ppm to give aromatized seamless capsules.

**[Table 6]**

| composition ratio | (-) |
|---|---|
| OMEGA-3-ACID ETYYL ESTERS 90 | 1000.0 |
| gelatin 4 | 364.5 |
| concentrated glycerin | 43.0 |
| sorbitol | 21.5 |
| purified water | 1287.0 |
| ethyl alcohol | 224.0 |
| blueberry flavor | 56.0 |

### Example 7

Gelatin 1 (jelly strength 245 g, viscosity 4.3 mPa·s, 3158 g), gelatin 2 (jelly strength 297 g, viscosity 5.3 mPa·s, 1579 g), concentrated glycerin (559.0 g), sorbitol solution (399.2 g, solid content: 299.4 g) and yellow dye No. 5 (1.17 g) were added to purified water (16610 g) heated to 52°C. The mixture was dissolved, and degassed under reduced pressure (aqueous shell composition solution). The composition ratio when OMEGA-3-ACID ETHYL ESTERS 90 is 1000.00 parts by weight is shown in Table 7.

An apparatus for seamless capsule production (SPHEREX, manufactured by Freund Corporation) was used to prepare seamless capsules. The temperature of "OMEGA-3-ACID ETHYL ESTERS 90" (capsule content) near the nozzle was controlled to 17.7 - 18.8°C, and the aqueous shell solution was controlled to 67.5 - 68.1°C. MCT oil was used as a carrier liquid and the temperature thereof was controlled to 6.4 - 6.8°C, and the mixture was encapsulated at a rate of 25 capsules per second.

The encapsulation step could be constantly operated stably by strictly controlling the temperatures.

The obtained capsules were cooled in a refrigerator (5°C, 21 hr), and dried in a drier until the water activity reached not more than 0.2. Then the carrier liquid on the capsule surface was wiped off to give seamless capsules containing 25 mg of OMEGA-3-ACID ETHYL ESTERS 90 per capsule.

The operation up to this point was repeated 3 times, and the obtained seamless capsules were mixed using a tumbler drier (manufactured by Freund Corporation). Ethyl alcohol (224 g) and blueberry flavor (56 g) were mixed to give a flavoring liquid. The flavoring liquid (199.1 g per 40655 g of seamless capsules) was dispersed on the mixed seamless capsules to aromatize them using a tumbler drier (manufactured by Freund Corporation). After aromatization, the ethyl alcohol in the seamless capsules was removed until it reached not more than 5000 ppm using a tumbler drier (manufactured by Freund Corporation) to give aromatized seamless capsules.

The size of the obtained capsules was about 4 mmφ (variation within the range of 3.5 - 4.5 mmφ) in the center value.

**[Table 7]**

| composition ratio | (-) |
|---|---|
| OMEGA-3-ACID ETYYL ESTERS 90 | 1000.00 |
| gelatin 1 | 242.94 |
| gelatin 2 | 121.47 |
| concentrated glycerin | 43.00 |
| sorbitol | 21.50 |
| yellow dye No. 5 | 0.086 |
| purified water | 1290.00 |
| ethyl alcohol | 224.0 |
| blueberry flavor | 56.0 |

The OMEGA-3-ACID ETHYL ESTERS 90 used in each of Examples 1 - 7 is a product compatible with the European Pharmacopoeia. The quality thereof is shown in Table 8.

**[Table 8]**

| | Ex. 1 | Ex. 2 | Ex. 3 | Ex. 4 | Ex. 5 | Ex. 6 | Ex. 7 |
|---|---|---|---|---|---|---|---|
| content rate of EPA ethyl ester | 45.3% w/w | 45.7% w/w | 45.3% w/w | 47.3% w/w | 47.0% w/w | 47.0% w/w | 47.1% w/w |
| content rate of DHA ethyl ester | 37.7% w/w | 37.6% w/w | 37.7% w/w | 36.9% w/w | 35.8% w/w | 35.8% w/w | 37.2% w/w |
| total content rate of EPA ethyl ester and DHA ethyl ester | 83.0% w/w | 83.3% w/w | 83.0% w/w | 84.2% w/w | 82.8% w/w | 82.8% w/w | 84.3% w/w |
| content rate of ω3-fatty acid alkyl ester | 92% w/w | 91% w/w | 92% w/w | 93% w/w | 91% w/w | 91% w/w | 92% w/w |
| content of α-tocopherol | 3.8 mg/g | 3.8 mg/g | 3.8 mg/g | 4.0 mg/g | 3.9 mg/g | 3.9 mg/g | 3.7 mg/g |
| European Pharmacopoeia | compatible | compatible | compatible | compatible | compatible | compatible | compatible |

| | | | | | | | |
|---|---|---|---|---|---|---|---|
| * The content rate and content in the table indicate the values in a capsule content (pharmaceutically active ingredient composition). | | | | | | | |

### Experimental Example 1

The seamless capsules (200 g) obtained in Example 3 were evaluated by a Digital Image Processing Particle Size Analyzer (Camsizer, manufactured by Horiba, Ltd.). The aspect ratio was not less than 0.95, which confirmed they were almost spherical.

### Experimental Example 2

In Example 3, as the content, OMEGA-3-ACID ETHYL ESTERS 90 could be contained at a high content of 100%. In addition, by capsule evaluation using a digital microscope (VHX-200, KEYENCE CORPORATION) before drying, it was observed that eyes were absent (which permits a conclusion that an eye having a size exceeding 1/2 of the average thickness of the shell was not present), and encapsulation with a good shell showing extremely less uneven thickness was achieved, as shown in Fig. 1. The thickness of the shell was 0.84 mm at maximum and 0.82 mm at minimum (minimum/maximum 0.98).

### Industrial Applicability

The seamless capsule of the present invention is superior in the stability of the capsule content, and can contain a large amount of the capsule content. Furthermore, the present invention affords high quality seamless capsules free of an eye or with only an extremely small eye even if present, which show extremely small nonuniformity in the thickness. Since the content can be high, the unit size of a preparation can be minimized, and highly easy administration and small space utility during storage can be realized. Moreover, the stability during preservation can be improved, thus enabling extension of a use-by date.

## Claims

1. A method of producing the seamless capsule comprising a shell formed by a shell composition comprising gelatin and a plasticizer, which does not comprise an interfacial tension modifier chosen in the group of phospholipid, fat, oils, surfactant, polar organic solvent and a gelling agent chosen in the group of polar organic solvents and polysaccharides, and a capsule content without comprising an interfacial tension modifier chosen in the group of phospholipid, fat, oils, surfactant, polar organic solvent and a gelling agent chosen in the group of polar organic solvents and polysaccharides by a drop in oil method, wherein temperatures of the "capsule content without comprising an interfacial tension modifier and a gelling agent", the "aqueous shell composition solution formed by a shell composition comprising gelatin and a plasticizer, which does not comprise an interfacial tension modifier and a gelling agent" and the "carrier liquid" are each controlled near multiple nozzles of an apparatus used for the drop in oil method.

2. The production method of claim 1, wherein the temperature of the "capsule content without comprising an interfacial tension modifier and a gelling agent" is controlled to a value set ± 2°C within the range of 5°C - 25°C, or the temperature of the "aqueous shell composition solution formed by a shell composition comprising gelatin and a plasticizer, which does not comprise an interfacial tension modifier and a gelling agent" is controlled to a value set ± 2°C within the range of 50°C - 80°C, or the temperature of the "carrier liquid" is controlled to a value set ± 1°C within the range of 1°C - 15°C, or a difference between the temperature of the "capsule content without comprising an interfacial tension modifier and a gelling agent" and the temperature of the "aqueous shell composition solution formed by a shell composition comprising gelatin and a plasticizer, which does not comprise an interfacial tension modifier and a gelling agent" is not less than 25°C and not more than 75°C, or a difference between the temperature of the "aqueous shell composition solution formed by a shell composition comprising gelatin and a plasticizer, which does not comprise an interfacial tension modifier and a gelling agent" and the temperature of the "carrier liquid" is not less than 35°C and not more than 79°C.

3. The production method of claim 1, wherein the capsule content is a pharmaceutically active ingredient composition, optionally the pharmaceutically active ingredient composition comprises ω3-fatty acid alkyl ester, optionally the pharmaceutically active ingredient composition comprises ω3-fatty acid alkyl ester in a proportion of not less than 90%w/w.

4. The production method of claim 3, wherein the pharmaceutically active ingredient composition comprises EPA ethyl ester (eicosapentaenoic acid ethyl ester) and DHA ethyl ester (docosahexaenoic acid ethyl ester) at a total ratio of the both components of not less than 80%w/w or comprises not less than 40%w/w of EPA ethyl ester and not less than 34%w/w of DHA ethyl ester, optionally the pharmaceutically active ingredient composition comprises OMEGA-3-ACID ETHYL ESTERS 90 in the European Pharmacopoeia.

5. A seamless capsule comprising a shell formed by a shell composition comprising gelatin and a plasticizer, which does notcomprise an interfacial tension modifier chosen in the group of phospholipid, fat, oils, surfactant, polar organic solvent and a gelling agent chosen in the *group of polar organic solvents and* polysaccharides*,* wherein the shell has a minimum/maximum thickness ratio of not less than 0.6, wherein the diameter of an eye of the shell after encapsulation and before drying is 1/2 or below of the average thickness of shells.

6. The seamless capsule of claim 5, wherein the shell is free of an eye.

7. The seamless capsule of claim 5, which does not comprise an interfacial tension modifier chosen in the group of phospholipid, fat, oils, surfactant, polar organic solvent and a gelling agent chosen in the group of polar organic solvents and polysaccharides*,* wherein the shell after encapsulation and before drying is free of an eye having a diameter exceeding 1/2 of the average thickness of shells.

8. The seamless capsule of claim 5, which comprises a capsule content free of an interfacial tension modifier chosen in the group of phospholipid, fat, oils, surfactant, polar organic solvent and a gelling agent chosen in the group of polar organic solvents and polysaccharides*.*

9. The seamless capsule of claim 5, wherein the capsule comprises a shell and a capsule content, wherein the capsule content is a pharmaceutically active ingredient composition, optionally the pharmaceutically active ingredient composition comprises ω3-fatty acid alkyl ester, optionally the pharmaceutically active ingredient composition comprises ω3-fatty acid alkyl ester in a proportion of not less than 90%w/w.

10. The seamless capsule of claim 9, wherein the pharmaceutically active ingredient composition comprises EPA ethyl ester (eicosapentaenoic acid ethyl ester) and DHA ethyl ester (docosahexaenoic acid ethyl ester) at a total ratio of the both components of not less than 80%w/w or comprises not less than 40%w/w of EPA ethyl ester and not less than 34%w/w of DHA ethyl ester, optionally the pharmaceutically active ingredient composition comprises OMEGA-3-ACID ETHYL ESTERS 90 in the European Pharmacopoeia.

## Patentansprüche

1. Verfahren zur Herstellung der nahtlosen Kapsel mit einer Hülle aus einer Gelatine und einen Weichmacher umfassenden Hüllenzusammensetzung, die keinen Grenzflächenspannungsmodifikator aus der Gruppe von Phospholipid, Fett, Ölen, Tensid, polarem organischem Lösungsmittel und kein Geliermittel aus der Gruppe polarer organischer Lösungsmittel und Polysaccharide umfasst, und mit einem Kapselinhalt, der keinen Grenzflächenspannungsmodifikator aus der Gruppe von Phospholipid, Fett, Ölen, Tensid, polarem organischem Lösungsmittel umfasst und kein Geliermittel aus der Gruppe polarer organischer Lösungsmittel und Polysaccharide umfasst, durch ein Tropfen-in-ÖI-Verfahren, wobei die Temperaturen des "Kapselinhalts ohne Grenzflächenmodifikator und Geliermittel", der "wässrigen Lösung der Hüllenzusammensetzung, die durch eine Gelatine und einen Weichmacher umfassende Hüllenzusammensetzung gebildet ist, die keinen Grenzflächenspannungsmodifikator und kein Geliermittel umfasst", und der "Trägerflüssigkeit" jeweils in der Nähe einer Vielzahl von Düsen einer für das Tropfen-in-ÖI-Verfahren verwendeten Vorrichtung geregelt werden.

2. Herstellungsverfahren nach Anspruch 1, wobei die Temperatur des "Kapselinhalts ohne Grenzflächenspannungsmodifikator und Geliermittel" auf einen Wert geregelt wird, der auf ± 2°C in dem Bereich von 5°C bis 25°C eingestellt ist, oder die Temperatur der "wässrigen Lösung der Hüllenzusammensetzung, die durch eine Gelatine und einen Weichmacher umfassende Hüllenzusammensetzung gebildet ist, die keinen Grenzflächenspannungsmodifikator und kein Geliermittel umfasst" auf einen Wert geregelt wird, der auf ± 2°C in dem Bereich von 50°C - 80°C eingestellt ist, oder die Temperatur der "Trägerflüssigkeit" auf einen Wert geregelt wird, der auf ± 1°C in dem Bereich von 1°C - 15°C eingestellt ist, oder eine Differenz zwischen der Temperatur des "Kapselinhalts ohne Grenzflächenspannungsmodifikator und Geliermittel" und der Temperatur der "wässrigen Lösung der Hüllenzusammensetzung, die durch eine Gelatine und einen Weichmacher umfassende Hüllenzusammensetzung gebildet wird, die keinen Grenzflächenspannungsmodifikator und kein Geliermittel umfasst" nicht weniger als 25°C und nicht mehr als 75°C beträgt, oder eine Differenz zwischen der Temperatur der "wässrigen Lösung der Hüllenzusammensetzung, die durch eine Hüllenzusammensetzung aus Gelatine und einem Weichmacher gebildet wird, die keinen Grenzflächenspannungsmodifikator und kein Geliermittel umfasst" und der Temperatur der "Trägerflüssigkeit" nicht weniger als 35°C und nicht mehr als 79°C beträgt.

3. Herstellungsverfahren nach Anspruch 1, wobei der Kapselinhalt eine pharmazeutische Wirkstoffzusammensetzung ist, die pharmazeutische Wirkstoffzusammensetzung optional ω3-Fettsäurealkylester umfasst, die pharmazeutische Wirkstoffzusammensetzung optional ω3-Fettsäurealkylester in einem Anteil von nicht weniger als 90 Gew.-% umfasst.

4. Herstellungsverfahren nach Anspruch 3, wobei die pharmazeutische Wirkstoffzusammensetzung EPA-Ethylester (Eicosapentaensäureethylester) und DHA-Ethylester (Docosahexaensäureethylester) in einem Verhältnis der beiden Komponenten von insgesamt nicht weniger als 80 Gew.-% umfasst oder nicht weniger als 40 Gew.-% EPA-Ethylester und nicht weniger als 34 Gew.-% DHA-Ethylester umfasst, optional umfasst die pharmazeutische Wirkstoffzusammensetzung OMEGA-3-SÄURE-ETHYLESTER 90 aus dem Europäischen Arzneibuch.

5. Nahtlose Kapsel mit einer Hülle, die durch eine Gelatine und einen Weichmacher umfassende Hüllenzusammensetzung gebildet wird, die keinen Grenzflächenspannungsmodifikator aus der Gruppe von Phospholipid, Fett, Ölen, Tensid, polarem organischem Lösungsmittel und kein Geliermittel aus der Gruppe von polaren organischen Lösungsmitteln und Polysacchariden umfasst, wobei die Hülle eine Verhältnis der kleinsten zur größten Dicke von nicht weniger als 0,6 besitzt, wobei der Durchmesser einer Öffnung der Hülle nach der Verkapselung und vor dem Trocknen maximal die Hälfte der durchschnittlichen Dicke der Hüllen beträgt.

6. Nahtlose Kapsel nach Anspruch 5, wobei die Hülle keine Öffnung besitzt.

7. Nahtlose Kapsel nach Anspruch 5, die keinen Grenzflächenspannungsmodifikator aus der Gruppe von Phospholipid, Fett, Ölen, Tensid, polarem organischem Lösungsmittel und kein Geliermittel aus der Gruppe polarer organischer Lösungsmittel und Polysaccharide umfasst, wobei die Hülle nach der Verkapselung und vor dem Trocknen keine Öffnung mit einem Durchmesser von mehr als der Hälfte der durchschnittlichen Dicke der Hüllen besitzt.

8. Nahtlose Kapsel nach Anspruch 5, die einen Kapselinhalt umfasst, der frei ist von einem Grenzflächenspannungsmodifikator aus der Gruppe von Phospholipid, Fett, Ölen, Tensid, polarem organischem Lösungsmittel und einem Geliermittel aus der Gruppe von polaren organischen Lösungsmitteln und Polysacchariden.

9. Nahtlose Kapsel nach Anspruch 5, wobei die Kapsel eine Hülle und einen Kapselinhalt umfasst, wobei der Kapselinhalt eine pharmazeutische Wirkstoffzusammensetzung ist, die pharmazeutische Wirkstoffzusammensetzung optional ω3-Fettsäurealkylester umfasst, die pharmazeutische Wirkstoffzusammensetzung optional ω3-Fettsäurealkylester in einem Anteil von nicht weniger als 90 Gew.-% umfasst.

10. Nahtlose Kapsel nach Anspruch 9, wobei die pharmazeutische Wirkstoffzusammensetzung EPA-Ethylester (Eicosapentaensäureethylester) und DHA-Ethylester (Docosahexaensäureethylester) in einem Verhältnis der beiden Komponenten von insgesamt nicht weniger als 80 Gew.-% umfasst oder nicht weniger als 40 Gew.-% EPA-Ethylester und nicht weniger als 34 Gew.-% DHA-Ethylester umfasst, optional umfasst die pharmazeutische Wirkstoffzusammensetzung OMEGA-3-SÄURE-ETHYLESTER 90 aus dem Europäischen Arzneibuch.

## Revendications

1. Procédé de production de la capsule sans soudure comprenant une enveloppe formée par une composition d'enveloppe comprenant de la gélatine et un plastifiant, qui ne comprend pas un agent modifiant la tension interfaciale choisi dans l'ensemble constitué par un phospholipide, une graisse, des huiles, un tensioactif, un solvant organique polaire, et un agent gélifiant choisi dans l'ensemble constitué par les solvants organiques polaires et les polysaccharides, et un contenu de la capsule ne comprenant pas un agent modifiant la tension interfaciale choisi dans l'ensemble constitué par un phospholipide, une graisse, des huiles, un tensioactif, un solvant organique polaire, et un agent gélifiant choisi dans l'ensemble constitué par les solvants organiques polaires et les polysaccharides, par un procédé à la goutte dans de l'huile, dans lequel les températures du « contenu de la capsule ne comprenant pas un agent modifiant la tension interfaciale et un agent gélifiant », de la « solution aqueuse de composition d'enveloppe formée par une composition d'enveloppe comprenant de la gélatine et un plastifiant, qui ne comprend pas un agent modifiant la tension interfaciale et un agent gélifiant » et du « liquide véhicule » sont réglées chacune au voisinage d'injecteurs multiples d'un appareil utilisé pour le procédé à la goutte dans de l'huile.

2. Procédé de production selon la revendication 1, dans lequel la température du « contenu de la capsule ne comprenant pas un agent modifiant la tension interfaciale et un agent gélifiant » est réglée à une valeur fixée à ± 2 °C dans la plage de 5 °C à 25 °C, ou la température de la « solution aqueuse de composition d'enveloppe formée par une composition d'enveloppe comprenant de la gélatine et un plastifiant, qui ne comprend pas un agent modifiant la tension interfaciale et un agent gélifiant » est réglée à une valeur fixée à ± 2 °C dans la plage de 50 °C à 80 °C, ou la température du « liquide véhicule » est réglée à une valeur fixée à ± 1 °C dans la plage de 1 °C à 15 °C, ou une différence entre la température du « contenu de la capsule ne comprenant pas un agent modifiant la tension interfaciale et un agent gélifiant » et la température de la « solution aqueuse de composition d'enveloppe formée par une composition d'enveloppe comprenant de la gélatine et un plastifiant, qui ne comprend pas un agent modifiant la tension interfaciale et un agent gélifiant » est d'au moins 25 °C et n'excède pas 75 °C, ou une différence entre la température de la « solution aqueuse de composition d'enveloppe formée par une composition d'enveloppe comprenant de la gélatine et un plastifiant, qui ne comprend pas un agent modifiant la tension interfaciale et un agent gélifiant » et la température du « liquide véhicule » est d'au moins 35 °C et n'excède pas 79 °C.

3. Procédé de production selon la revendication 1, dans lequel le contenu de la capsule est une composition de composants pharmaceutiquement actifs, en option la composition de composants pharmaceutiquement actifs comprend un ester alkylique d'acide gras ω3, en option la composition de composants pharmaceutiquement actifs comprend un ester alkylique d'acide gras ω3 en une proportion d'au moins 90 % p/p.

4. Procédé de production selon la revendication 3, dans lequel la composition de composants pharmaceutiquement actifs comprend de l'ester éthylique d'EPA (ester éthylique d'acide eicosapentaénoïque) et de l'ester éthylique de DHA (ester éthylique d'acide docosahexaénoïque) en une proportion totale des deux composants d'au moins 80 % p/p ou comprend au moins 40 % p/p d'ester éthylique d'EPA et au moins 34 % p/p d'ester éthylique de DHA, en option la composition de composants pharmaceutiquement actifs comprend des ESTERS ÉTHYLIQUES D'ACIDES OMÉGA-3 90 dans la Pharmacopée européenne.

5. Capsule sans soudure comprenant une enveloppe formée par une composition d'enveloppe comprenant de la gélatine et un plastifiant, qui ne comprend pas un agent modifiant la tension interfaciale choisi dans l'ensemble constitué par un phospholipide, une graisse, des huiles, un tensioactif, un solvant organique polaire, et un agent gélifiant choisi dans l'ensemble constitué par les solvants organiques polaires et les polysaccharides, dans laquelle l'enveloppe à un rapport d'épaisseurs minimale/maximale d'au moins 0,6, dans laquelle le diamètre d'un « oeil » de l'enveloppe après encapsulation et avant séchage est égal à 1/2 ou moins de l'épaisseur moyenne des enveloppes.

6. Capsule sans soudure selon la revendication 5, dans laquelle l'enveloppe est exempte d'un « oeil ».

7. Capsule sans soudure selon la revendication 5, qui ne comprend pas un agent modifiant la tension interfaciale choisi dans l'ensemble constitué par un phospholipide, une graisse, des huiles, un tensioactif, un solvant organique polaire, et un agent gélifiant choisi dans l'ensemble constitué par les solvants organiques polaires et les polysaccharides, dans laquelle l'enveloppe après encapsulation et avant séchage est exempte d'un « oeil » ayant un diamètre excédant 1/2 de l'épaisseur moyenne des enveloppes.

8. Capsule sans soudure selon la revendication 5, qui comprend un contenu de la capsule exempt d'un agent modifiant la tension interfaciale choisi dans l'ensemble constitué par un phospholipide, une graisse, des huiles, un tensioactif, un solvant organique polaire, et d'un agent gélifiant choisi dans l'ensemble constitué par les solvants organiques polaires et les polysaccharides.

9. Capsule sans soudure selon la revendication 5, où la capsule comprend une enveloppe et un contenu de la capsule, le contenu de la capsule étant une composition de composants pharmaceutiquement actifs, en option la composition de composants pharmaceutiquement actifs comprend un ester alkylique d'acide gras ω3, en option la composition de composants pharmaceutiquement actifs comprend un ester alkylique d'acide gras ω3 en une proportion d'au moins 90 % p/p.

10. Capsule sans soudure selon la revendication 9, dans laquelle la composition de composants pharmaceutiquement actifs comprend de l'ester éthylique d'EPA (ester éthylique d'acide eicosapentaénoïque) et de l'ester éthylique de DHA (ester éthylique d'acide docosahexaénoïque) en une proportion totale des deux composants d'au moins 80 % p/p ou comprend au moins 40 % p/p d'ester éthylique d'EPA et au moins 34 % p/p d'ester éthylique de DHA, en option la composition de composants pharmaceutiquement actifs comprend des ESTERS ÉTHYLIQUES D'ACIDES OMÉGA-3 90 dans la Pharmacopée européenne.
